Europäisches Patentamt

European Patent Office

Office européen des brevets

(11) Veröffentlichungsnummer: **0 141 912**
A1

(12) **EUROPÄISCHE PATENTANMELDUNG**

(21) Anmeldenummer: 84109001.2

(22) Anmeldetag: 30.07.84

(51) Int. Cl.⁴: **A 61 M 5/28**, A 61 M 5/18

(30) Priorität: 24.08.83 CH 4808/83

(43) Veröffentlichungstag der Anmeldung: 22.05.85
Patentblatt 85/21

(84) Benannte Vertragsstaaten: **BE DE FR GB IT NL**

(71) Anmelder: **Holzner, Günter Wolfgang, Chemin des Palettes 15, CH-1212 Grand-Lancy (CH)**

(72) Erfinder: **Holzner, Günter Wolfgang, Chemin des Palettes 15, CH-1212 Grand-Lancy (CH)**

(54) **Elastischer, transparenter Hohlköper für flexible Injektionspackungen.**

(57) Gegenstand der Erfindung ist ein Hohlkörper (6) aus elastischem, transparentem Kunststoffmaterial für flexible, vorgefüllte oder leere Injektionspackungen (1). Er befindet sich zwischen der Kanüle (1) und der Auslassöffnung der Injektionspackung (1) und erlaubt es durch Zusammendrükken unmittelbar vor dem Einstechen und Loslassen nach erfolgter Penetration einige Tropfen Blut anzusaugen und so die richtige Lage der Kanülenspitze im Inneren des Blutgefässes zu kontrollieren. Nach dem gleichen Prinzip ist diese Vorrichtung auch geeignet entsprechende Mengen von Blut oder einer anderen Flüssigkeit aus dem Körper anzusaugen.

ACTORUM AG

0141912

## Elastischer, transparenter, Hohlkörper für flexible, gebrauchsfertige Injektionspackungen

Gegenstand der Erfindung ist ein Hohlkörper aus elastischem, transparentem Kunststoffmaterial als Bestandteil von flexiblen, gebrauchsfertigen Injektionspackungen. Besagter Hohlkörper befindet sich zwischen der Kanüle und der Auslassöffnung der Injektionspackung. Vor dem Einstechen der Kanüle wird der elastische Hohlkörper mit den Fingerspitzen oder einer mechanischen Vorrichtung zusammengedrückt. Befindet sich die Kanülenspitze im Inneren der Vene oder Arterie lässt man den besagten Hohlkörper los. Dieser ist dank seiner Elastizität bestrebt sein normales Volumen wieder zu erreichen und saugt dadurch einige Tropfen Blut aus der penetrierten Ader. Das reibungslose Ansaugen des Blutes ist der Beweis für eine richtig ins Innere der Ader eingeführte Kanülenspitze und entspricht dem zu demselben Zweck üblichen Aspirieren mit dem Kolben einer normalen Injektions Spritze. Der beschriebene elastische Hohlkörper kann auch nach demselben Prinzip eine seiner Grösse entsprechende Menge von Blut oder einer anderen Körperflüssigkeit ansaugen die dann für weitere Untersuchungen zur Verfügung steht.

## Stand der Technik

Die derzeit für zahlreiche Injektionen oder zur Blutentnahme verwendeten Einmalspritzen, meisst aus Kunststoff oder Glas hergestellt, haben prinzipiell den gleichen Aufbau und die gleiche Funktion wie die seit Langem bekannten, sterilisierbaren und oftmals verwendbaren Injektionsspritzen. Die Injektionslösung wird in einer eigenen Ampulle aufbewahrt die vor Gebrauch geöffnet wird um die Injektionslösung in die Spritze zu saugen.
Injektionskanüle und Spritze sind meist getrennt verpackt und werden vor Gebrauch zusammengesetzt. Dieses Verfahren erfordert einige Handgriffe die, falls nicht sachkundig ausgeführt, die Gefahr einer Infektion der Injektionslösung oder Kanüle in sich bergen.

Gebrauchsfertige Injektionspackungen die die Injektionsflüssigkeit bereits vorgefüllt und sterilisiert enthalten
weisen diesen Nachteil nicht auf. In den letzten Jahren
wurden einige Injektionspackungen patentiert die im Prinzip
aus einem vorgefüllten Plastikbeutel bestehen der an einem
Ende mit der Kanüle in Verbindung steht. Durch Druck auf
die Aussenwände des Beutels mit den Fingerspitzen oder
einer entsprechenden zangenartigen Vorrichtung wird die
Injektionsflüssigkeit durch die Kanüle gepresst und
injiziert.

Genauere Beschreibungen der verschiedenen Ausführungen
sind in den folgenden Patentschriften zu finden :
G.W. Holzner : Schweizer Patentgesuch Nr. 2 767/81-5, 1981
V.C. Hall : US Patent 3,114,369, 1963
B.G. Szabo : US Patent 3,736,933, 1973
W.F. Van Eck : US Patent 4,130,117, 1978

Ein Nachteil aller dieser Injektionspackungen ist es, dass
es nicht möglich ist, nach Einstich in eine Vene oder
Arterie zu kontrollieren, ob die Spitze der Kanüle sich
richtig im Inneren des Blutgefässes befindet. Bei den
derzeit allgemein üblichen Injektionsspritzen wird der
Kolben leicht zurückgezogen (Aspirieren) wodurch, bei
richtiger Lage der Kanülenspitze, einige Bluttropfen in
die Spritze gesaugt werden.

Gegenstand der vorliegenden Erfindung ist ein Hohlkörper
aus elastischem Kunststoff der sich zwischen der Auslassöffnung der vorhin beschriebenen, vorgefüllten Injektionsbeutel und der Kanüle befindet. Er ermöglicht die Kontrolle
der richtigen Lage der Kanülenspitze im Inneren des Blutgefässes bei flexiblen Injektionssystemen und das Ansaugen
einer seiner Grösse entsprechenden Menge an Blut oder einer
anderen Körperflüssigkeit.

0141912

## Die Erfindung

Gegenstand der Erfindung ist ein elastischer, transparenter Hohlkörper aus Kunststoff der zwischen der Austrittsöffnung einer flexiblen Injektionspackung und der Kanüle angebracht ist.

Das Volumen des besagten Hohlkörpers liegt zwischen 0,1 - 500 ml. Seine äussere Form ist so gestaltet, dass man den Hohlkörper zwischen Daumen und Zeigefinger einer Hand oder mit einer mechanischen Vorrichtung leicht zusammendrücken kann. Vor dem Einstechen der Kanüle wird der besagte Hohlkörper vollständig zusammengedrückt. Die Kanüle wird sodann wie üblich in das Blutgefäss eingeführt und erst dann wird der Hohlkörper losgelassen so dass er dank seiner Elastizität wieder seine ursprüngliche Form annimmt. Durch den dabei entstehenden Saugeffekt wird eine geringe Menge Blut aus dem Inneren des penetrierten Blutgefässes in den transparenten Hohlkörper gesaugt. Ein anstandsloses Ansaugen des Blutes ist nur dann möglich wenn die Kanülenspitze sich richtig im Inneren der Ader befindet und kann daher als Kontrolle für die richtige Durchführung einer intravenösen oder intraarteriellen Injektion dienen. Nun erfolgt das Einspritzen der Injektionslösung durch Zusammenpressen des gefüllten Injektionsbeutels.
Zur Blutentnahme wird ein entsprechend grösserer elastischer Hohlkörper verwendet der das Blut ansaugt und in sich aufnimmt.

Der elastische Hohlkörper hat noch weitere Vorteile : will man mit der gleichen einmal eingeführten Nadel mehrere Lösungen injizieren, so ist es nötig die Injektionspackung vom Nadelhalter abzusetzen und eine neue Packung anzuschliessen. Ein einfaches Zusammendrücken des elastischen Hohlkörpers der an der Nadel verbleibt genügt, um das Einsaugen von Luft oder das Austreten von arteriellem Blut während des Wechselns zu verhindern.

Eine oder mehrere anschliessende Injektionen können auch
dadurch erfolgen, dass man den an der in der Ader eingeführten Nadel verbleibenden elastischen Hohlkörper direkt
mit der Nadel der anderen Injektionspackungen ansticht und
die Injektionsflüssigkeit in den Hohlkörper injiziert. Die
Injektionsflüssigkeit gelangt dadurch über die bereits eingeführte Nadel ins Blut.
Der elastische Hohlkörper kann auch über ein Ventil verfügen
das das Einfliessen oder die Entnahme von Flüssigkeiten
gestattet.

0141912

## Zeichenerklärung für fig. 1-10

fig. 1   Injektionspackung mit Ueberdruckventil und elastischem
         Hohlkörper, Seitenansicht.

fig. 2   Injektionspackung mit aufreissbarer Verschlussnaht und
         elastischem Hohlkörper, Seitenansicht.

fig. 3   wie fig. 2 jedoch Aufsicht.

fig. 4   Injektionspackung mit aufreissbarer Verschlussnaht
         eingebaut im elastischen Hohlkörper.

fig. 5   Injektionspackung wie fig. 2 und 3 jedoch mit einem
         flexiblen Verlängerungsschlauch zwischen Injektionspackung und elastischem Hohlkörper.

fig. 6   Elastischer Hohlkörper zur Entnahme grösserer
         Flüssigkeitsmengen aus dem Körper.

fig. 7   Elastischer Hohlkörper zur Entnahme von kleinen
         Blut- oder Flüssigkeitsmengen.

fig. 8   Elastischer Hohlkörper zur Entnahme von kleinen Blut-
         oder Flüssigkeitsmengen mit Verlängerungsschlauch
         zwischen Nadel und Hohlkörper.

fig. 9   Wie fig. 8 jedoch mit Verlängerungsschlauch und
         Verschlussventil nach dem Hohlkörper.

fig. 10  Elastischer Hohlkörper zur Entnahme von kleinen
         Blut- oder Flüssigkeitsmengen mit fester Bodenplatte.

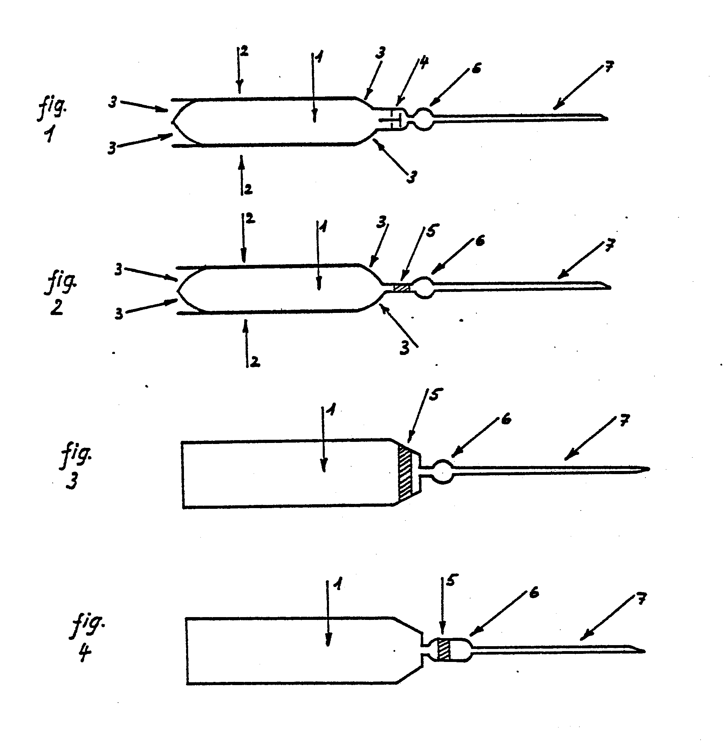

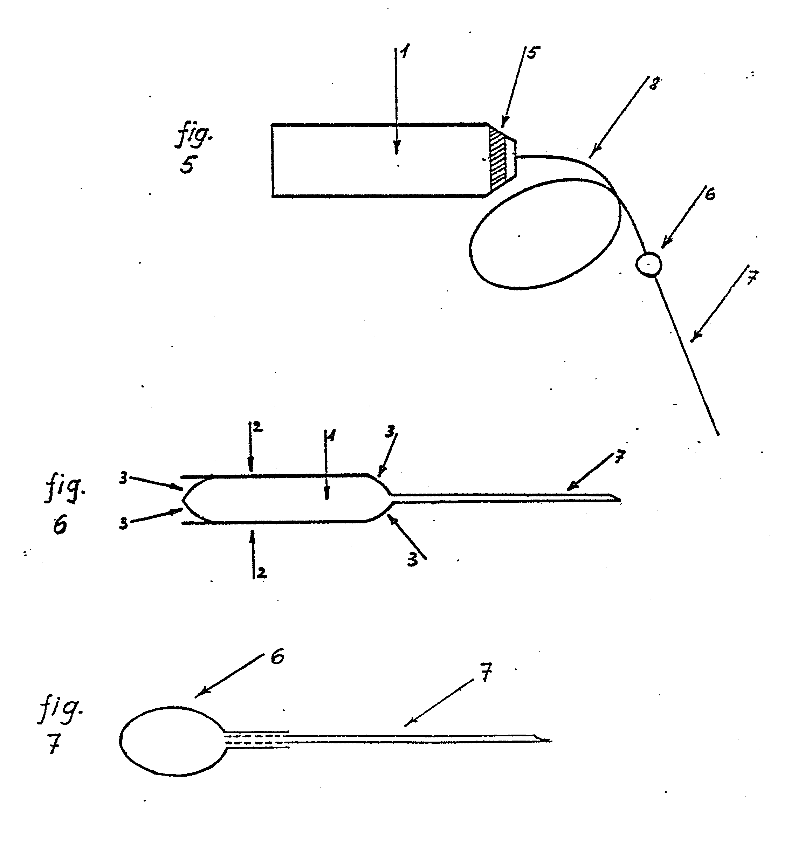

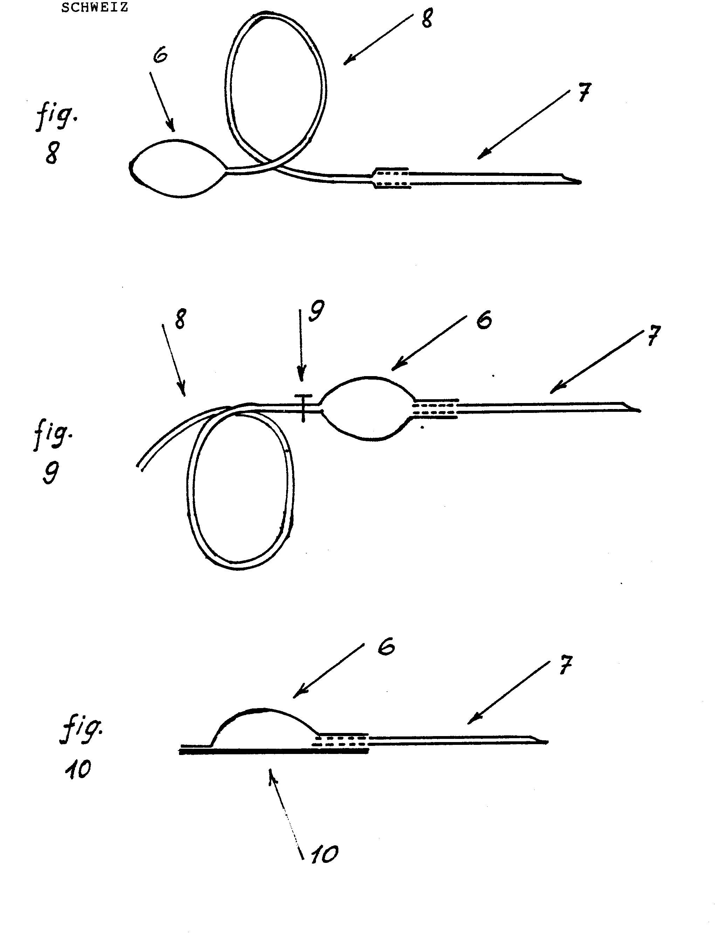

### Erklärung der Einzelteile für fig. 1-10

1. Kunststoffbeutel
2. Steife Seitenwand
3. Flexible Teile
4. Ueberdruckventil
5. Aufreissbare Schweissnaht
6. Elastischer, transparenter Hohlkörper
7. Kanüle
8. Flexibler Verlängerungsschlauch
9. Verschlussventil
10. Feste Bodenplatte

Patentansprüche

1. Gebrauchsfertige Injektionspackung bestehend aus einem verschlossenen, vollständig zusammendrückbaren Kunststoffbeutel der mit der Injektionslösung gefüllt oder leer ist und an einem Ende in Verbindung mit einer Injektionskanüle steht dadurch gekennzeichnet, dass sich zwischen besagtem Kunststoffbeutel und Injektionskanüle ein elastischer, transparenter Hohlkörper aus Kunststoff befindet.

2. Injektionspackung nach Anspruch 1 dadurch gekennzeichnet, dass der besagte elastische Hohlkörper ein Volumen von 0,1 - 500 ml hat.

3. Injektionspackung nach Anspruch 1 und 2 dadurch gekennzeichnet, dass der elastische Hohlkörper eine Form aufweist die es erlaubt denselben vor Einführung der Kanüle in ein Blutgefäss oder in einen anderen Hohlraum des Körpers mit den Fingerspitzen oder einer mechanischen Vorrichtung vollständig zusammenzudrücken.

4. Injektionspackung nach Anspruch 1, 2 und 3 dadurch gekennzeichnet, dass der besagte elastische, zusammengedrückte Hohlkörper beim Loslassen einige Tropfen Blut aus der entsprechenden Ader ansaugt und so die richtige Penetration der Vene oder Arterie durch die Kanülenspitze anzeigt.

5. Injektionspackung nach Anspruch 1, 2 und 3 dadurch gekennzeichnet, dass der elastische, zusammengedrückte Hohlkörper entsprechend seinem Volumen eine grössere Menge von Blut oder einer anderen Körperflüssigkeit ansaugt und in seinem Inneren ansammelt.

- 2 -

0141912

6. Injektionspackung nach Anspruch 1, 2, 3 und 5 dadurch gekennzeichnet, dass der elastische, zusammendrückbare Hohlkörper mit Reagenzien vorgefüllt ist die zur Analyse oder Konservierung von Blut oder einer anderen Körperflüssigkeit dienen.

Gunter W. Holzner
Chemin des Palettes 15
CH - 1212  Grand-Lancy
SCHWEIZ

1/3

Zeichnungen fig 1 - 4
0141912

fig. 1

fig. 2

fig. 3

fig. 4

fig.
5

fig.
6

fig.
7

fig. 8

fig. 9

fig. 10

Europäisches
Patentamt

# EUROPÄISCHER RECHERCHENBERICHT

## EINSCHLÄGIGE DOKUMENTE

EP 84109001.2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 3 089 489 (R.P. DUNMIRE) | 1-5 | A 61 M 5/28 |
| Y | * Gesamt; insbesondere Spalte 3, Zeilen 9 bis 45 * | 6 | A 61 M 5/18 |
| | -- | | |
| Y | DE - A1 - 2 605 044 (H. WELLER) | 6 | |
| | * Seite 9, 3. Absatz bis Seite 10, 1. Absatz; Seite 14, 2. Absatz * | | |
| | -- | | |
| Y | DE - A1 - 3 032 018 (F. HUTTERER) | 6 | |
| | * Patentansprüche 1 und 2 * | | |
| | -- | | |
| D,A | US - A - 3 736 933 (B.G. SZABO) | 1-5 | |
| | * Gesamt * | | RECHERCHIERTE SACHGEBIETE (Int. Cl.4) |
| | -- | | |
| D,A | US - A - 4 130 117 (W.F. VAN ECK) | 1-5 | A 61 M |
| | * Gesamt * | | A 61 B |
| | ---- | | |

Der vorliegende Recherchenbericht wurde für alle Patentansprüche erstellt.

| Recherchenort | Abschlußdatum der Recherche | Prüfer |
|---|---|---|
| WIEN | 31-01-1985 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82